# EUROPÄISCHE PATENTSCHRIFT

(11) **EP 0 879 577 B1**
(45) Veröffentlichungstag und Bekanntmachung des Hinweises auf die Patenterteilung: **26.10.2005**
(21) Anmeldenummer: 98810284.4
(22) Anmeldetag: 02.04.1998
(51) Int. Cl.: A61B 17/16

(54) **Fräser für medizinische Zwecke**
Milling cutter for medical purposes
Fraise pour usage médical

(30) Priorität: 22.05.1997 CH 19697
(43) Veröffentlichungstag der Anmeldung: 25.11.1998
(73) Patentinhaber: PRECIMED S.A., 2534 Orvin (CH)
(72) Erfinder: Da Rold, Orlando, 4500 Solothurn (CH)
(74) Vertreter: Bugnion Genève

(56) Entgegenhaltungen:
- EP-A- 0 733 343
- US-A- 3 939 497
- US-A- 4 116 200

## Beschreibung

Die vorliegende Erfindung betrifft einen Fräser für medizinische Zwecke insbesondere für den Einsatz bei orthopädischen Operationen, der einen Fräskörper aus dünnwandigem Material aufweist, je nach Einsatzzweck geformt ist, und auf seiner Oberfläche der Fräsergrösse und Fräserform in Anzahl und Position systematisch zugeordnete Fräszähne aufweist, wobei die Anordung und Form der Fräszähne mit industriell üblichen Genauigkeiten definiert sind.

Fräser oder Raffelfräser der vorerwähnten Art sind an sich bekannt. Man findet sie auch unter der Bezeichnung "Formraspeln oder Raspeln". In der Chirurgie werden solche Werkzeuge vor allem bei orthopädischen Operationen eingesetzt. Sie dienen dazu den Knochen formgerecht auszufräsen, d.h. einen eigentlichen Implantatsitz vorzubereiten, damit ein einzusetzendes Implantat möglichst guten Halt findet. Zu diesem Zweck werden verschiedenste Fräser eingesetzt, deren Form dem jeweiligen Einsatzzweck entsprechend vorgegeben ist.

Einen Raffelfräser wie er beispielhaft für den Einsatz in Hüftgelenkpfannen verwendet wird, stellt z.B. WO 95/13479 vor. Eine weit verbreitete Form der Fräszähne wird z.B. in EP 0733 343 vorgestellt. Raffelfräser für Hüftgelenkpfannen mit Fräszähnen wie sie in oben erwähnter Veröffentlichung vorgestellt werden, fertigt man in der Regel nach dieser Methode: Auf einer Metallscheibe der entsprechenden Dicke werden die Positionen der Fräszähne angezeichnet. Diese Scheibe wird dann z.B. durch Tiefziehen oder Pressen in die gewünschte Form gebracht. Im nächsten Arbeitsgang am dreidimensionalen Prüfling wird an den Stellen, die vorher angezeichnet wurden, jeweils eine Oeffnung gestanzt. Diese Oeffnung wird angesenkt, um eine Schneidkante zu erzeugen. Darauf wird die Oeffnung mit einem Dorn aufgedrückt, um die gewünschte Form eines Fräszahnes mit Schneide und Zahngrube zu erhalten.

Der Fräskörper weist nach dem Tiefziehen oder Pressen eine räumliche Form auf. Dies macht es ausserordentlich aufwendig, die Zähne auf herkömmlichen Werkzeugmaschinen herzustellen. Investition und Aufwand zum Einrichten der Werkzeuge stehen in keinem Verhältnis zum Ertrag. Die Operationen Stanzen, Ansenken und mit einem Dorn Aufdrücken werden deshalb in der Regel von Hand durchgeführt. Die Qualität ist dadurch die für Handarbeit typische. Die Oeffnungen für die Fräszähne werden nach Augenmass im Bereich der vorgängig angezeichneten Positionen gestanzt. Eine gute Masshaltigkeit der Dimensionen von Fräszahn und Zahngrube nach dem Stanzen, Ansenken und Aufdrücken kann deshalb niemand gewährleisten. Jeder auf diese Art gefertigte Fräser ist ein Unikat.

Die herkömmlichen Herstellungsverfahren zeichnen sich denn auch dadurch aus, dass man in der Fertigung viel Ausschuss ausscheiden muss. Die Formen der Fräser und der Fräszähne weisen grosse Ungenauigkeiten auf. Zudem sind die Fräszähne im allgemeinen stumpf, so dass der Chirurg Kraft anwenden muss, um die gewünschte Form- und Massgenauigkeit im zu bearbeitenden Bereich des Knochen zu erreichen. Bedingt durch die Ungenauigkeit der Formen von Fräser und Fräszähnen, werden zwei Fräser gleicher Dimension unterschiedliche Implantatsitze im Knochengut erzeugen. Es ist von der Geschicklichkeit des Chirurgen und der Qualität des verwendeten Fräsers abhängig, ob der vorbereitete Implantatsitz im Knochengut für die Aufnahme des Implantates die richtige Grösse und Form aufweist.

Es wäre jedoch wünschbar, dass der Chirurg durch Wahl des richtigen Fräsers die Sicherheit hat, dass der zu fräsende Implantatsitz im Knochengut das industriell gefertigte Implantat möglichst formschlüssig aufnimmt.

Die vorliegende Erfindung stellt sich nunmehr die Aufgabe, für den Fräszahn eine Form zu finden, die es zulässt, einen Fräser der eingangs genannten Art derart zu verbessern, dass Fräser und Fräszähne, mit Massgenauigkeiten von Hundertstel Millimeter reproduzierbar und in Industriequalität, hergestellt werden können. Ferner muss der Chirurg ohne grossen Kraftaufwand in die Lage versetzt werden, präzise Implantatsitze im Knochenbereich vorbereiten zu können.

Diese Aufgabe löst ein Fräser mit den Merkmalen des Patentanspruches 1. Weitere erfindungsgemässe Merkmale gehen aus den abhängigen Ansprüchen hervor und deren Vorteile sind in der nachfolgenden Beschreibung erläutert.

In der Zeichnung zeigt:
- Figur 1: Aufsicht auf einen erfindungsgemässen Fräser
- Figur 2: Seitenansicht eines erfindungsgemässen Fräsers
- Figur 3: Aufsicht auf einen Fräszahn
- Figur 4: Schnittansicht eines Fräszahnes durch Linie A-A
- Figur 5: Aufsicht auf einen Fräszahn

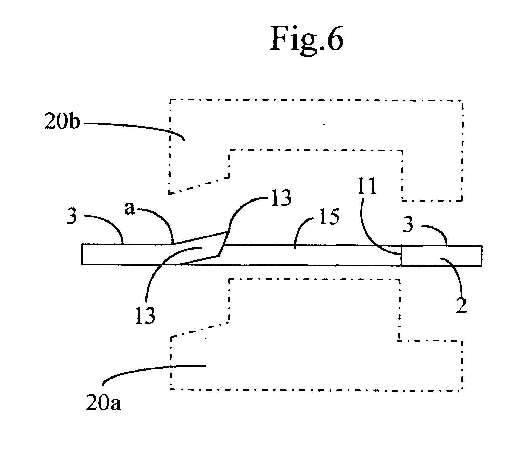

- Figur 6: Schnittansicht eines Fräszahnes durch Linie A-A, mit Werkzeugen

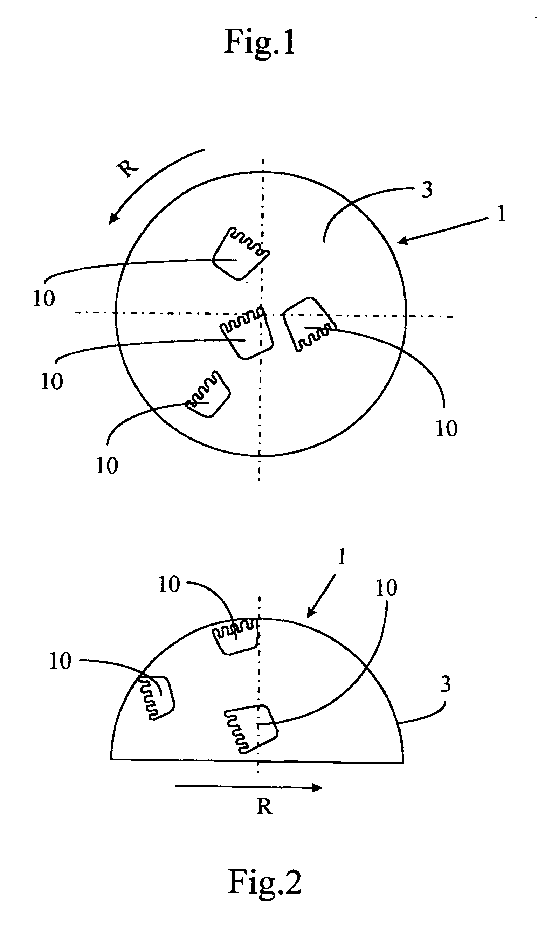

Eine mögliche Ausführung des erfindungsgemässen Fräsers 1 zeigen z.B. Fig. 1 und Fig. 2. Mit dem Fräser 1 muss ein Implantatsitz gleicher Form aus dem Knochenbereich herausgearbeitet werden können. Dies kann zum Beispiel bei einem Acetabulum-Raffelfräser nur erreicht werden, wenn der Fräskörper 2 eine formgetreue Halbkugel ist, die Fräszähne 10 an der richtigen Stelle im Fräskörper 2 angeordnet sind, einheitliche Dimensionen aufweisen und gut schneiden.

Damit der Fräskörper 2 seine Form behält, die er durch Tiefziehen oder Pressen erhalten hat, darf er bei allen nachfolgenden Bearbeitungsgängen nicht mehr verformt werden. Dies wird gemäss Erfindung erreicht, indem man die Form eines Fräszahnes 10 so wählt, dass maschinelle Fertigungsmethoden eingesetzt werden können und die Positionen der Fräszähne mit Methoden der CAD und CAM Technik definiert. Die Fräszähne 10 können so durch die Maschine ohne vorheriges Anzeichen in den Fräskörper 2 eingearbeitet werden. Moderne Schneidmethoden lassen dies durch moderne CIM- und CAM-Technik ohne weiteres zu.

Durch die genaue Definition der Position der Fräszähne 10 auf dem Fräskörper 2 erreicht man eine Optimierung der Anzahl von Fräszähnen 10 die notwendig sind. Es leuchtet ein, dass die Gefahr der Verformung des Fräskörpers 2 kleiner ist, je weniger Fräszähne eingearbeitet werden müssen. Viele Fräszähne 10 schwächen zudem die Stabilität des Fräskörpers 2. Aus diesen Gründen ist wünschbar, möglichst wenig Fäszähne 10 auf dem Fräskörper 2 anbringen zu müssen. Andererseits ist die Reduktion von Fräszähnen 10 nur dann möglich, wenn deren Dimensionen genau und reproduzierbar, so dass ihr Schneidverhalten voraussehbar ist.

Die Herstellung erfolgt in drei Arbeitsgängen: Im ersten Arbeitsgang werden die Rohlinge aus Blech mittels Tiefziehen oder Pressen in die gewünschte Form des Fräskörpers 2 gebracht. Aus dem massgenauen Fräskörper 2 werden dann im zweiten Arbeitsgang auf einer gesteuerten Maschine die Oeffnungen 15, mit massgenauer Bezugskante 11 und scharfer Schneide 13 mit scharfer Schnittkante 18 herausgearbeitet. Dies geschieht mit modernen Methoden der Schneid- und Fertigungstechnik. Brauenbildung und Verformung des Fräskörpers 11 sind kein Problem und selbst schwer zu bearbeitende Materialien können mit der notwendigen Präzision bearbeitet werden. In einem dritten Arbeitsgang wird die Schneidezunge 13 um Winkel α um die Biegekante a aufgebogen, damit die Schneide 12 in die richtige Lage gebracht wird. Dieser Arbeitsgang wird in vielen Fällen in Handarbeit erfolgen.

Um diese Handarbeit zu erleichtern, soll die Form der Oeffnung 15 so definiert sein, dass sie einem Werkzeug 20a als Führung dienen kann. Dazu wird die ganze Form oder einzelne Elemente der Oeffnung 15 definiert, dimensioniert und bearbeitet. Sie kann z.B. durch Schneidezunge 13, Zwischenräume 14, seitliche Begrenzungen 16 und die, der Schneidezunge 13 gegenüberliegende Bezugskante 11 definiert werden. Die erforderliche Führung für ein Werkzeug 20a kann auch dadurch erreicht werden, dass z.B. die Bezugskante 11 und die Zwischenräume 14 genau definiert sind. Diese Methode hat den Vorteil, dass Schneidezunge 13 und seitliche Begrenzungen 16 freier gestaltet werden können. Für die Spanabfuhr durch die Oeffnung 15, für die dieselbe im Eisatz ebenfalls eine Funktion übernehmen muss, können daraus Vorteile entstehen.

In der Fertigung dient die Oeffnung 15 oder Elemente davon als Lehre und Führung für den Werkzeugteil 20a. Die Bezugskante 11 dient in jedem Fall der Positionierung der Biegekante a. Diese beiden Elemente werden durch Distanz und Position in Abhängigkeit zueinander definiert. Liegt der Werkzeugteil 20a für das Aufbiegen der Schneidzungen 13 an der Bezugskante 11 an, wird die Linie a automatisch in der vorgesehenen Lage zur Bezugskante 11 zu liegen kommen. Die seitliche Führung des Werkzeugteiles 20a wird entweder durch die Zwischenräume 14, die seitlichen Begrenzungen 16, oder die Seitenkanten 17 der Schneidzungen 13 definiert.

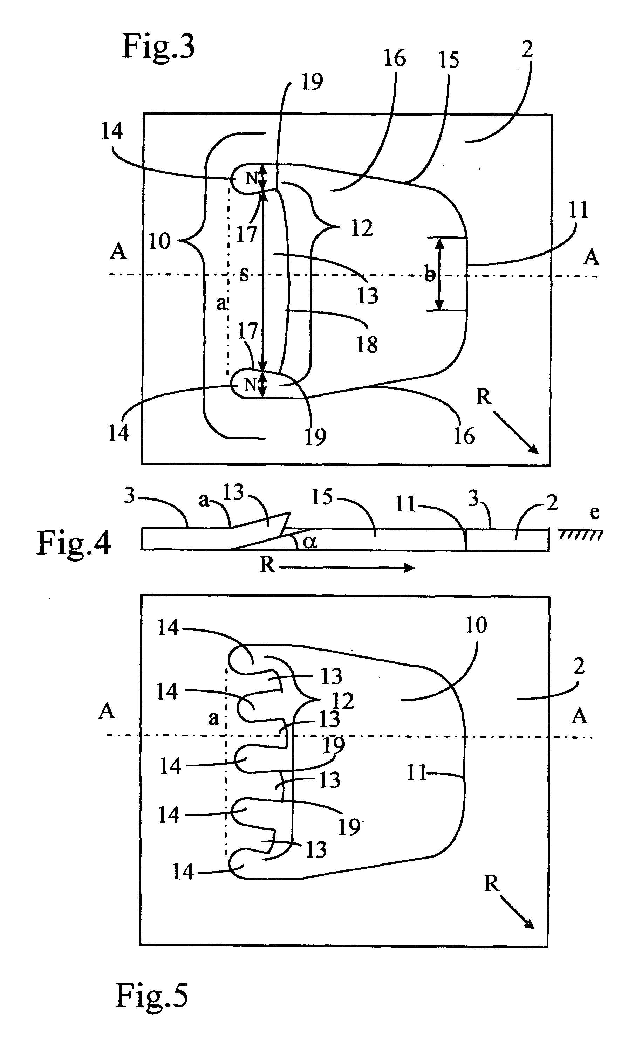

Nun wird der Fräskörper 2 so über den unteren Teil eines Werkzeuges 20a senkrecht zur Ebene e des Fräszahnes 10 zu liegen kommen (siehe Fig. 4). In dieser Stellung wird der oberen Werkzeugteil 20b von Hand oder durch eine Maschine niedergedrückt. Die beiden Werkzeugteile 20a und 20b bringen zusammengepresst die Schneidezunge 13 in die vorgesehene Lage.

Solche Fräszähne 10 können in beliebigen Formen von Fräskörpern 2 angebracht werden. Werden die Fräszähne 10 in ein flaches Blech als Fräskörper 2 eingearbeitet, erhält man eine Art Feile oder Raspel mit der beliebige Formen bearbeitet werden können. Solche Fräser dienen z.B. dem Formen eines Knochenendstückes. Die kugelige Form des Fräskörpers 2 ist oben beschrieben und wird vornehmlich für die Vorbereitung des Implantatsitzes für Hüftgelenkprothesen verwendet. Zylindrische Fräskörper 2 können z.B. zur Vorbereitung des Implantatsitzes im Oberschenkelknochen für Hüftprothesen dienen. Wie die kugelige Pfanne, die im Beckenknochen Halt finden muss, soll auch der Prothesenschaft, welcher in den Oberschenkelknochen eingepasst wird, fest sitzen. Die Qualität des Sitzes ist auch bei dieser Anwendung von der Qualität des verwendeten Werkzeugs abhängig.

Die Form der Schneide 12 spielt in den Anwendungen eine wichtige Rolle. Sie kann wie in Fig. 1 gezeigt aus einer Schneidzunge 13 gebildet werden. Fig. 3 zeigt die Möglichkeit, dass mehrere Schneidzungen 13 die Schneide 12 bilden. Ein spezieller Effekt wird dadurch erreicht, dass man den Fräszahn 10 im Einsatz in einer zur Achse A-A abweichender Richtung R wie auf Fig. 3 gezeigt, bewegt. In diesem Falle ist die in Bewegungsrichtung R liegende Ecke 19 eines Fräszahnes 10 zuerst im Eingriff.

Es ist von Bedeutung, aus welchen Elementen der Schneidezunge 13 die Schneide 12 gebildet wird. Naheliegend ist das Schärfen der Schnittkante 18, so dass nur diese Schnittkante 18 die Schneide 12 bildet. Für einfache Fräser 1 die vornehmlich in der Richtung der Achse A-A bewegt werden, mag dies genügen. In vielen anderen Fällen ist es wichtig, dass die Schneide 12 nicht nur durch die Schnittkante 18 gebildet wird, sondern auch die Seitenkanten 17 und die Ecken 19 der Schneidzungen geschärft sind, so dass Seitenkanten 17, Schnittkanten 18 und Ecken 19 gemeinsam die Schneide 12 bilden.

## Patentansprüche

1. Fräser (1) für medizinische Zwecke insbesondere für den Einsatz bei orthopädischen Operationen, der einen Fräskörper (2) aus dünnwandigem Material aufweist, je nach Einsatzzweck geformt ist und auf seiner Oberfläche (3) der Fräsergrösse und Fräserform in Anzahl und Position systematisch zugeordnete Fräszähne (10) aufweist, wobei die Anordnung und Form der Fräszähne (10) mit industriell üblichen Genauigkeiten definiert sind, **dadurch gekennzeichnet, dass** die Fräszähne (10) eine Oeffnung (15) in der Oberfläche (3) eines Fräskörpers (2) bilden, wobei diese Oeffnung (15) aus mindestens einer Schneidezunge (13), zwei Zwischenräumen (14), zwei seitlichen Begrenzungen (16) und einer Bezugskante (11) gebildet wird, wobei die Bezugskante (11) mit der Länge 1b gegenüber der Schneide (12) angeordnet ist und eine kleinere Länge aufweist, als die Summe der Länge 1s der Schneide (12) und der Breite 1z der Zwischenräume (14).

2. Fräser für medizinische Zwecke nach Anspruch 1, **dadurch gekennzeichnet, dass** der Fräserkörper (2) ein flaches Blech ist.

3. Fräser für medizinische Zwecke nach Anspruch 1, **dadurch gekennzeichnet, dass** der Fräskörper (2) eine kugelige Form aufweist.

4. Fräser für medizinische Zwecke nach Anspruch 1, **dadurch gekennzeichnet, dass** der Fräskörper (2) eine zylindrische Form aufweist.

5. Fräser für medizinische Zwecke nach Anspruch 1, **dadurch gekennzeichnet, dass** der Fräskörper (2) eine beliebige räumliche Form aufweist.

6. Fräser für medizinische Zwecke nach Anspruch 1, **dadurch gekennzeichnet, dass** die Schneide (12) durch die Schnittkante (18) der Schneidezunge (13) gebildet wird.

7. Fräser für medizinische Zwecke nach Anspruch 1, **dadurch gekennzeichnet, dass** die Schneide (12) durch Schnittkante (1) und Seitenkanten (17) gebildet wird.

## Claims

1. A milling cutter (1) for medical purposes, in particular for use in orthopaedic operations, which has a milling body (2) made of thin-walled material, is shaped in accordance with the intended application and on its surface (3) has milling teeth (10) systematically assigned in terms of number and position to the milling cutter size and milling cutter shape, the arrangement and shape of the milling teeth (10) being defined with the level of accuracy which is industrially conventional, **characterized in that** the milling teeth (10) form an opening (15) in the surface (3) of a milling body (2), this opening (15) being formed from at least one cutting tongue (13), two gaps (14), two lateral boundaries (16) and a reference edge (11), the reference edge (11) having the length 1b being arranged opposite the cutter (12) and having a shorter length than the total on the length 1s of the cutter (12) and the width 1z of the gaps (14).

2. The milling cutter for medical purposes according to claim 1, wherein the milling cutter body (2) is a planar metal sheet.

3. The milling cutter for medical purposes according to claim 1, wherein the milling cutter body (2) has a spherical form.

4. The milling cutter for medical purposes according to claim 1, wherein the milling cutter body (2) has a cylindrical form.

5. The milling cutter for medical purposes according to claim 1, wherein the milling cutter body (2) has any desired three-dimensional form.

6. The milling cutter for medical purposes according to claim 1, wherein the cutter (12) is formed by the cutting edge (18) of the cutting tongue.

7. The milling cutter for medical purposes according to claim 1, wherein the cutter (12) is formed by a cutting edge (18) and side edges (17).

## Revendications

1. Fraise (1) à usage médical, en particulier pour un usage lors d'opérations orthopédiques, qui comporte un corps de fraise (2) en matériau à paroi mince, formé en fonction de l'application et comprenant sur sa surface (3) des dents de fraisage (10) arrangées en nombre et en position en fonction de la taille de la fraise et de sa forme, l'arrangement et la forme des dents de fraisage (10) étant définie par des tolérances habituelles dans l'industrie, **caractérisé en ce que** les dents de fraisage (10) forment une ouverture (15) dans la surface du corps de la fraise (2), cette ouverture (15) étant formée d'au moins une lame tranchante (13), de deux interstices (14), de deux bordures latérales (16) et d'une arête de référence (11), l'arête de référence avec une longueur lb étant placée en face du tranchant (12) et ayant une longueur inférieure à la somme de la longueur ls du tranchant (12) et de la largeur lz des interstices (14).

2. Fraise pour usage médical selon la revendication 1, **caractérisée en ce que** le corps de la fraise (2) est une tôle plane.

3. Fraise pour usage médical selon la revendication 1, **caractérisée en ce que** le corps de la fraise (2) a une forme sphérique.

4. Fraise pour usage médical selon la revendication 1, **caractérisée en ce que** le corps de la fraise (2) a une forme cylindrique.

5. Fraise pour usage médical selon la revendication 1, **caractérisée en ce que** le corps de la fraise (2) a une forme tridimensionnelle quelconque.

6. Fraise pour usage médical selon la revendication 1, **caractérisée en ce que** le tranchant (12) est formé par l'arête de coupe (18) de la lame (13).

7. Fraise pour usage médical selon la revendication 1, **caractérisée en ce que** le tranchant (12) est formé par l'arête de coupe (18) et par les arêtes latérales (17).
